Europäisches Patentamt

European Patent Office

Office européen des brevets

(19)

(11) Publication number: **0 053 800**
**B1**

(12) **EUROPEAN PATENT SPECIFICATION**

(45) Date of publication of patent specification: **18.06.86**

(21) Application number: **81110101.3**

(22) Date of filing: **03.12.81**

(51) Int. Cl.⁴: **C 12 P 19/04,** A 61 K 31/715
// C12R1/20

(54) Heteroglycan, process for the production of same, and immunological activator containing same.

(30) Priority: **06.12.80 JP 171473/80**

(43) Date of publication of application:
**16.06.82 Bulletin 82/24**

(45) Publication of the grant of the patent:
**18.06.86 Bulletin 86/25**

(84) Designated Contracting States:
**AT BE CH DE FR GB IT LI LU NL SE**

(56) References cited:

CHEMICAL ABSTRACTS, vol. 82, no. 19, 12th
May 1975, page 410, no. 123329q, Columbus
Ohio (USA);

CHEMICAL ABSTRACTS, vol. 71, no. 25, 22nd
December 1969, page 114, no. 120726d,
Columbus Ohio (USA); M.LEMBO: "Effects of
temperature on growth and chemical
composition of a psychrophilic bacteria"

CHEMICAL ABSTRACTS, vol. 94, no. 23, 8th
June 1981, page 309, no. 188402a, Columbus
Ohio (USA); M. FLETCHER: "The question of
passive versus active attachment mechanisms
in nonspecific bacterial adhesion"

(73) Proprietor: **MICROBIAL CHEMISTRY RESEARCH
FOUNDATION
14-23, Kamiosaki 3 Chome Shinagawa-ku
Tokyo 141 (JP)**

(72) Inventor: **Umezawa, Hamao, Prof.
4-23, Toyotama Kita Nerima-ku
Tokyo (JP)**
Inventor: **Okami, Yoshiro, Dr.
4-18-14, Denenchofu Ohta-ku
Tokyo (JP)**
Inventor: **Kurasawa, Shogo, Dr.
5-39-2, Sendagi Bunkyo-ku
Tokyo (JP)**
Inventor: **Ohnuki, Takashi, Dr.
1155-2, Nakamaruko Nakahara-ku
Kawasaki City Kanagawa Prefecture (JP)**

(74) Representative: **Meyer-Dulheuer, Karl-Hermann,
Dr. et al
HOECHST Aktiengesellschaft Zentrale
Patentabteilung Postfach 80 03 20
D-6230 Frankfurt/Main 80 (DE)**

Courier Press, Leamington Spa, England.

## Description

### Background of the invention

The present invention relates to a heteroglycan, a process for the production of the same, and an immunological activator containing the same.

A number of polysaccharides have heretofore been discovered, and it is known that they have a wide variety of uses.

It has now been found that a specific microorganism produces a novel heteroglycan, and the novel heteroglycan has immunopotentiating activity.

### Brief description of the drawings

Fig. 1 is a gel filtration chromatogram of heteroglycan which is obtainable by a ®Sepharose CL4B column (1.6 cmφ×80 cm, 5 milliliters of 0.5N-NaCl solution containing 2 milligrams of sample II per milliliter), eluted with 0.5N-NaCl solution at a flow rate of 7 milliliters per hour, and fractionated the eluate into 3 milliliter fractions:

Fig. 2 is a gel filtration chromatogram of heteroglycan which is obtained by a ®Sepharose CL4B column (φ 16×340 mm, 0.5 milliliter of 0.05M-NaCl solution containing 0.5 milligram of Sample III per milliliter) eluted with 0.05M-NaCl solution at a flow rate of 20 milliliters per hour, and fractionated the eluate into 0.8 milliliter fractions:

Fig. 3 is a gel filtration chromatogram of heteroglycan which is obtained by the same manner except that a ®Sepharose CL2B column was used instead of a ®Sepharose CL4B column.

Fig. 4 is an infrared absorption spectrum of heteroglycan of the invention.

### Detailed description of the invention

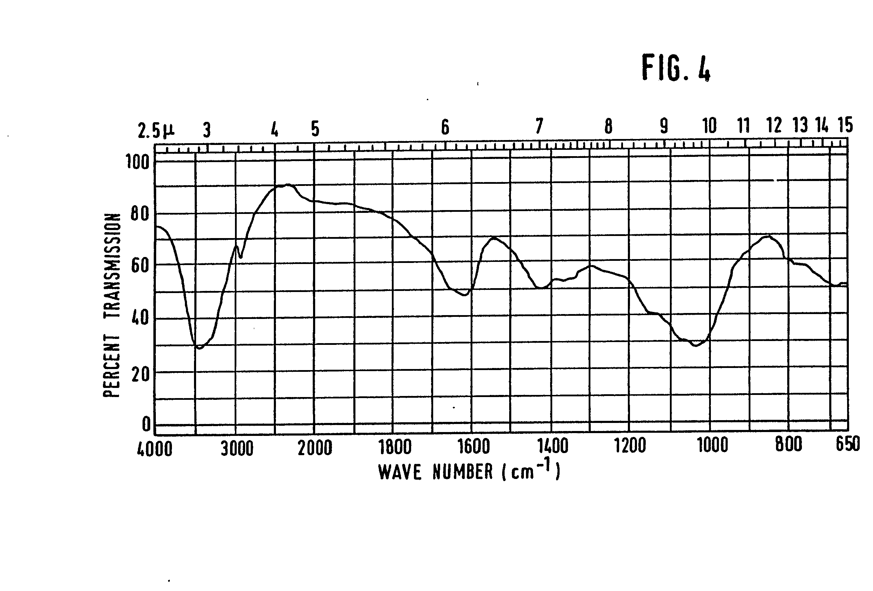

The heteroglycan of this invention is composed of (a) fucose, (b) mannose and (c) glucose in the molar ratio of (a):(b):(c)=0.15±0.05:0.31±0.05:1 and has an infrared absorption spectrum as shown in Fig. 4. The heteroglycan is in the fermentation broth of a microorganism belonging to the genus Flavobacterium and can be obtained by recovering therefrom.

The heteroglycan of the invention is produced by a cultivation of a microorganism belonging to the genus Flavobacterium having an ability to produce the heteroglycan in a medium consisted of carbon sources, nitrogen sources, inorganic ions, and if necessary, organic compounds such as vitamins and amino acids, in sea water (including artificial sea water), and the heteroglycan produced and accumulated in the fermentation broth is separated and recovered.

Any carbon sources which are usually used for the cultivation of microorganisms can be used in the invention. Preferred examples of such carbon sources are carbohydrates such as glucose, sucrose, starch and dextrin. Also, nitrogen sources which are usually used for the cultivation of microorganisms can be used in the invention. Examples of such nitrogen sources include peptone, yeast extract, meat extract, corn steep liquor, soybean powder, casein, and ammonium ions.

Cultivation is performed preferably under aerobic conditions with stirring. The cultivation temperature can be appropriately selected within the range that the heteroglycan-producing microorganism can grow and produce the heteroglycan, and it is usually preferred to be from 10°C to 35°C and more preferably from 24°C to 30°C. The cultivation is performed until the desired heteroglycan is sufficiently accumulated in the fermentation broth. Usually, the cultivation time is from 15 hours to 100 hours.

The microorganism capable of producing the heteroglycan of the present invention is *Flavobacterium uliginosum* MP-55 (FERM-P 5793).

The strain is collected and isolated from marine environment and has the following microbiological characteristics:

(I) Morphological characteristics

(1) Form and size of the cell: Rod and 0.2 to 0.3 μm×0.6 to 0.9 μm

(2) Pleomorphism: Non-pleomorphic

(3) Motility: Non-motile

(4) Sporogenicity: Non-sporulating

(5) Gram's stain: Negative

(6) Acid-fastness: Negative

(II) Culture characteristics on various media

(1) Bouillon agar plate culture: No growth

(2) Marine agar 2216 medium (produced by Difco) plate culture: Medium growth, circular, flat to semilenticular, entire margin, smooth, glistening, translucent, mucoid, and yellow to yellow orange

(3) Bouillon agar slant culture: No growth

(4) Marine agar 2216 culture (produced by Difco) slant culture: Moderate growth, membranous, thread-like, glistening, and yellow to orange

(5) Bouillon liquid culture: No growth

(6) Marine 2216 medium (produced by Difco) liquid culture: Slightly turbid, and no membrane is formed on the surface

2

(7) Bouillon gelatin stab culture: No changes
(8) Marine 2216 medium (produced by Difco) gelatin stab culture: Liquefaction
(9) Litmus milk: No changes.

(III) Physiological characteristics
(1) Reduction of nitrates: Positive
(2) Denitrification: Negative
(3) MR test: Negative
(4) VP test: Negative
(5) Production of indole: Negative
(6) Production of hydrogen sulfide: Negative
(7) Hydrolysis of starch: Positive
(8) Utilization of citric acid: Negative on a Koser medium, and negative on a Christensen medium
(9) Utilization of inorganic nitrogen source: Utilizes nitrates and ammonium salts
(10) Production of dye: No formation of water-soluble dye
(11) Urease: Negative
(12) Oxidase: Positive
(13) Catalase: Positive
(14) Growth ranges: Temperature: Good growth at 8°C to 35°C; optimum temperature: 24°C to 30°C; and no growth at 37°C. pH: 6 to 9
(15) Attitude toward oxygen: Aerobic
(16) O—F Test (according to the Hugh & Leifson method): No acid is formed
(17) Production of acids and gases from saccharides (basic culture composition: 0.5% peptone, 2% NaCl, 1% $MgCl_2$, 0.2% $CaCl_2$, 0.1% KCl, and 0.02% BCP):

| Saccharides | Production of acid | Production of gas |
|---|---|---|
| L-Arabinose | − | − |
| D-Xylose | + | − |
| D-Glucose | + | − |
| D-Mannose | − | − |
| D-Fructose | − | − |
| D-Galactose | − | − |
| Maltose | + | − |
| Sucrose | + | − |
| Lactose | − | − |
| Trehalose | − | − |
| D-Sorbitol | − | − |
| D-Mannitol | − | − |
| Inositol | − | − |
| Glycerin | − | − |
| Starch | − | − |
| Raffinose | − | − |
| Cellobiose | + | − |
| D-Ribose | − | − |
| Rhamnose | + | − |

3

(18) Salt resistance: Growth on Marine 2216 medium (produced by Difco) with addition of 2% NaCl, but no growth with addition of 4% NaCl

(19) Nutrient requirement: No growth occurs unless sea water is added.

(IV) DNA-GC Content

36.3% (according to the Tm method).

Summarizing the above described microbial characteristics of the strain for use in the invention with reference to Bergey's Manual of Determinative Bacteriology, 8th Ed., it has been determined that the present strain belongs to the genus Flavobacterium. Furthermore, as a result of a comparative study of the present strain with the known strains described in the above Bergey's Manual of Determinative Bacteriology, it has been found that the known strain most similar to the present strain is Flavobacterium uliginosum.

The present strain is in agreement with *Flavobacterium uiliginosum* in respect of: color tone of colony, no growth at 37°C, no high salt resistance, liquefaction of gelatin, no growth on a medium containing no sea water, reduction of nitrates, and production of acids from glucose, sucrose, and maltose. On the other hand, they are different from each other, for example, in respect of production of acid from lactose and DNA-GC content. These differences, however, are believed to be negligible since no key to distinguish the species has been determined.

At present, therefore, it is believed suitable to determine that the present strain is not a novel strain, but *Flavobacterium uliginosum*, and the present strain has been identified as *Flavobacterium uliginosum* ZoBell and Upham 1944. The present strain has been deposited in the Fermentation Research Institute, Agency of Industrial Science and Technology, Ministry of International Trade and Industry of Japan under the accession number of FERM-P 5793.

Mutants obtained by irradiation with ultraviolet rays, X rays, or treatment with chemicals, etc. can be used in the present invention as long as they have the ability to produce the heteroglycan of the present invention.

The heteroglycan of this invention can be recovered from the fermentation broth by known methods. For example, the fermentation broth is subjected to centrifugal separation to provide a supernatant liquid. Ethanol is added to the supernatant liquid, the resulting mixture is allowed to stand in an ice chamber to cause precipitation, the precipitate is isolated, extracted with hot water, and is subjected to centrifugal separation to provide a supernatant liquid, the supernatant liquid is dialyzed, ethanol is then added thereto to cause precipitation, and the resulting precipitate is dried to obtain a crude heteroglycan.

The crude heteroglycan thus obtained is then further purified. For this purpose, various methods can be used. For example, chloroform and n-butanol are added to an aqueous solution of the crude heteroglycan, and the resulting mixture is stirred and is then subjected to centrifugal separation to obtain a supernatant liquid. The same procedure as above is applied repeatedly once to several times to the supernatant liquid. To the supernatant liquid thus obtained is added with ethanol to precipitate a purified heteroglycan. A 0.5N-NaCl solution of the heteroglycan is passed through a ®Sepharose CL4B column and eluted with 0.5N-NaCl at a suitable flow rate. The eluate is fractionated in an appropriate amount, and heteroglycan-containing fractions are collected and dialyzed to remove NaCl therefrom. Then, ethanol is added to cause precipitation whereby a purified heteroglycan fraction can be obtained.

The physical and chemical properties of the heteroglycan (MP-55 substance) of this invention are described below:

The sugar content of the purified heteroglycan fraction obtained by the above gel filtration determined by the phenol sulfuric acid method (assuming that the water content is 10 to 20%) is 75 to 85% calculated as glucose, and the protein content determined by the Lowry method is 0 to 0.4%.

One milligram of the heteroglycan is reacted with 2 milliliters of 5% hydrogen chloride-methanol at 90°C for 20 hours to cause methanolysis. Then, an excessive amount of silver carbonate is added to neutralize excess hydrochloric acid in the reaction solution, and the resulting insoluble materials are removed. Methanol is then distilled away. The reaction mass is dried overnight under reduced pressure in the presence of phosphorus pentaoxide, and is trimethylsilylated with trimethylsilylating reagent to be subjected to gas chromatography. Gas chromatographic analysis with reference to a standard sample shows that the heteroglycan of this invention is composed of fucose, mannose and glucose. The sugar composition is shown in the table below.

TABLE 1

|  | Example 1 (%) | Example 2 (%) | Example 3 (%) |
|---|---|---|---|
| Fucose | 11.0 | 11.6 | 10.0 |
| Mannose | 18.6 | 19.2 | 21.0 |
| Glucose | 70.4 | 69.2 | 69.0 |

4

# 0 053 800

The molar ratio of (a) fucose, (b) mannose and (c) glucose is determined to be (a):(b):(c)=0.15±0.05:0.31±0.05:1.

One example of elemental analysis of the heteroglycan of this invention is: C: 37.43%; H: 5.61%; ash content: 5.53%; and water content: 16.3%. The water content varies depending on humidity.

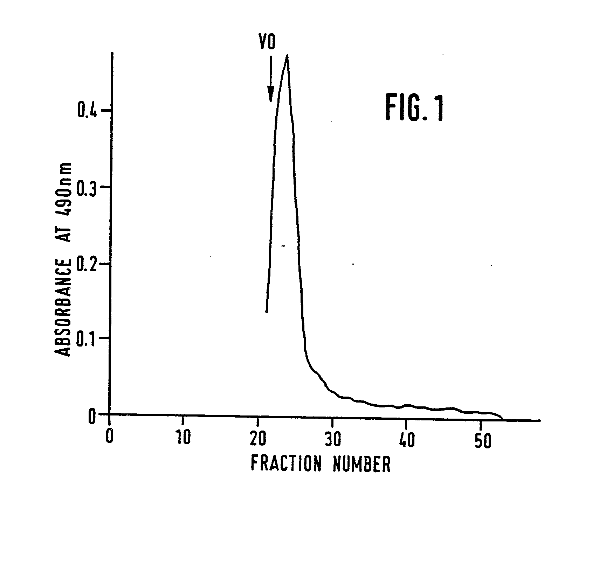

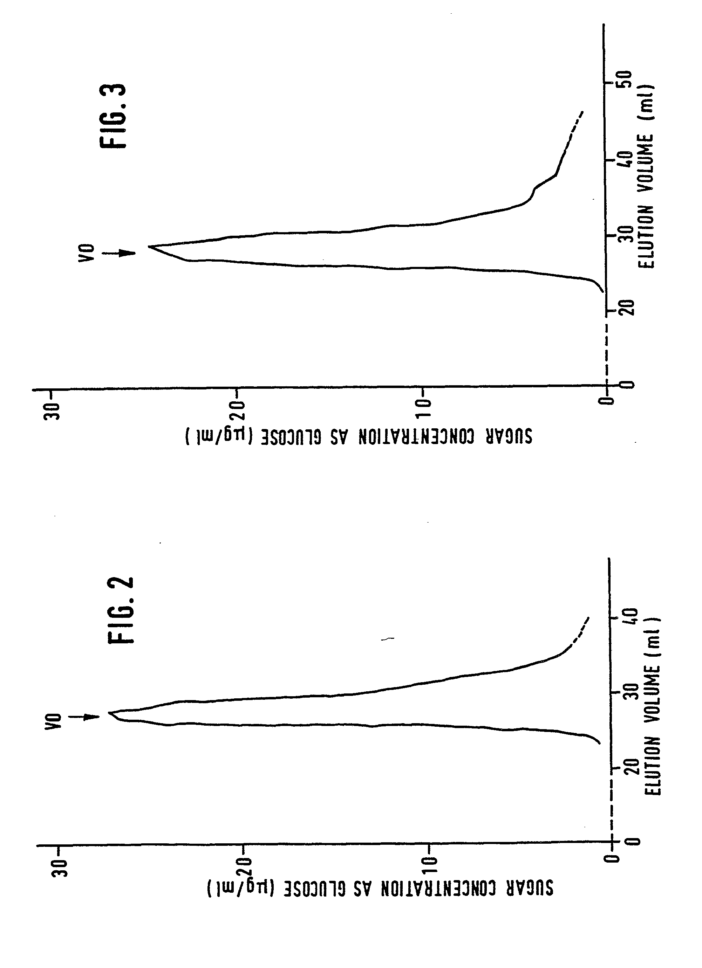

The molecular weight of the heteroglycan of this invention determined by a gel filtration method (the method of Fig. 1) is 500,000 or more. Further, the molecular weight of the heteroglycan of this invention is 5,000,000 or more when it is determined by a gel filtration method (the method of Fig. 2) and is 20,000,000 or more when it is determined by a gel filtration method (the method of Fig. 3). Thus, molecular weight of the heteroglycan is varied with the conditions of Employed.

The optical rotation is $[\alpha]_{589}^{22}=+69°$ (c=0.13, $H_2O$).

The infrared absorption spectrum employing the KBr tablet is shown in Fig. 4. The ultraviolet absorption spectrum shows no maximum absorption.

The heteroglycan is white, and an aqueous solution thereof is neutral. The heteroglycan is insoluble in organic solvents, but is soluble in water, NaCl solution and alkaline solutions. The heteroglycan is positive in the anisaldehyde-sulfuric acid reaction and phenol-sulfuric acid reaction, and is negative in the ninhydrin reaction.

The heteroglycan of the present invention has very strong immunopotentiating activity, and it is therefore useful for prevention and treatment of infectious diseases. In addition, the heteroglycan exhibits the antitumor effect mainly mediated by immunological response of host.

Hereinafter this invention is described in detail with reference to the following Examples.

Example 1

*Flavobacterium uliginosum* MP-55 strain (FERM-P 5793) was inoculated in one liter of a medium (pH 7.2) of artificial sea water (Jamarin S, produced by Jamarin Laboratory) containing 0.6% glucose, 0.5% polypeptone and 0.1% yeast extract, and incubated with shaking at 27°C for two days.

The fermentation broth was collected and was subjected to centrifugal separation to obtain a supernatant. An equal volume of ethanol to the above liquid was added and the resulting mixture was allowed to stand overnight in an ice chamber to cause precipitation. The precipitate thus formed was filtered and extracted with 100 milliliters of hot water. This extraction procedure was repeated and, thereafter, centrifugal separation was again applied to obtain a supernatant liquid. The supernatant was dialyzed overnight against running water, and 150 milliliters of ethanol was added thereto. The precipitate thus obtained was dried under reduced pressure to obtain 170 milligrams of a crude heteroglycan (hereinafter referred to as "Sample I").

In order to remove protein impurities contained in Sample I, a 1/3 volume of chloroform and 1/30 volume of n-butanol were added to a 2 grams of Sample I per liter of aqueous solution and the resulting mixture was vigorously stirred for 30 minutes and was subjected to centrifugal separation at 3,000 r.p.m. for 15 minutes to obtain a supernatant liquid. The above procedure was repeated twice (three times if includes a previous procedure). Then, an equal volume of ethanol to the supernatant liquid was added to yield a purified heteroglycan (hereinafter referred to as "Sample II").

Five milliliters of a 0.5N-NaCl solution containing 2 milligrams of Sample II per milliliter was passed through a ®Sepharose CL4B (1.6 centimeter φ×80 centimeters, 0.5N-NaCl), eluted with 0.5N-NaCl solution at a flow rate of 7 milliliters per hour, and the eluate was fractionated into 3 milliliter fractions. Fraction A shown in Fig. 1 was collected and dialyzed to remove NaCl. Then, ethanol was added to precipitate a purified heteroglycan fraction (hereinafter referred to as "Sample III").

The sugar and protein contents of each sample were measured, and the results are shown in Table 2 below.

TABLE 2

| Sample | Sugar content (%)[*1] | Protein content (%)[*2] |
|---|---|---|
| I | 60 to 80 | 8 to 15 |
| II | 70 to 85 | 1 to 3 |
| III | 75 to 85 | 0 to 0.4 |

[*1]: According to the phenol-sulfuric acid method. Calculated as glucose.
[*2]: According to the Lowry method.

Sample III had the physical and chemical properties as described hereinbefore.

Example 2

Using the same strain and medium as used in Example 1, cultivation in tank fermentor was performed at 27°C for 18 hours under the conditions with an aeration amount of 1/2 v.v.m. and agitation of 300 r.p.m. The same procedure as in Example 1 was applied to provide 327 milligrams per liter of a crude

5

heteroglycan (hereinafter referred to as "Sample IV"). Sample IV was purified by the deproteinization procedure as described in Example 1 to obtain a purified heteroglycan (hereinafter referred to as "Sample V").

Example 3
Antitumor test

(a) To ICR mice (5 weeks old, female) were transplanted subcutaneously $3 \times 10^6$ of Sarcoma-180. For 10 days from the day subsequent to the transplantation, an appropriate amount of sample was intraperitoneally administered. At 5th week, the weight of tumor was measured to determine the antitumor activity of the sample. The results are shown in Table 3 below.

TABLE 3

| Sample | Administration amount (milligrams per kilogram×day) | Number of mice | Tumor inhibitory ratio (%) | Rate of complete regression* |
|---|---|---|---|---|
| II | 5×10 | 7 | 74 | 2/7 |
|  | 20×10 | 7 | 85 | 2/7 |
| V | 5×10 | 7 | 95 | 3/7 |
|  | 20×10 | 7 | 92 | 2/6 |

\* Number of mice completely freed from notable tumor/number of survived mice.

(b) The effect of prolonging the survival time by administration of each sample was measured. To seven ICR mice (5 weeks old, female) were intraperitoneally transplanted Sarcoma-180 ascitic tumor in a proportion of $1 \times 10^6$ cells per mouse. For 10 days from the day subsequent to the transplantation, the sample was intraperitoneally administered in a proportion of 25 milligrams per kilogram. The average number of survival days and life-prolonging effect were determined. The results are shown in Table 4.

Also, antitumor activity of the Sample on the Sarcoma-180 solid tumor in nude mice was tested in a proportion of 25 milligrams per kilogram to thereby determine the tumor inhibitory activity by intraperitoneal administration of the sample. The results are shown in Table 5.

TABLE 4

| Sample | Administration amount (milligrams per kilogram×day) | Average number of survival days (days) | Increase of life span (%) |
|---|---|---|---|
| Control | — | 15.5±5.7 | 100 |
| I | 25×10 | 27.8±22.0 | 179.3 |
| II | 25×10 | 33.6±23.8 | 216.8 |

TABLE 5

| Sample | Administration amount (milligrams per kilogram×day) | Weight of tumor (grams) | Tumor-inhibitory ratio (%) |
|---|---|---|---|
| Control | — | 10.7±5.8 | — |
| II | 25×10 | 4.7±2.4 | 56 |

(c) to C3H/He mice was transplanted subcutaneously mouse mammary carcinoma MM102 in a proportion of $3 \times 10^6$ cells per mouse. At appropriate days subsequent to the transplantation, the sample was administered in a ratio of 50 milligrams per kilogram, and the antitumor effect was determined. The results are shown in Table 6.

### TABLE 6

| Days of administration | Administration method | Tumor inhibitory ratio (%)* | Average of survival days (days) | Increase of life span (%) |
|---|---|---|---|---|
| Control | — | — | 64.1±21.0 | 100 |
| Continuously for 10 days | Intraperi- toneously | 51.8 | 77.6±18.0 | 121.1 |
| At 10th and 14th days | Intratumorly | 59.8 | 77.5±21.5 | 117.8 |

* Measured at 5th week.

In order to determine the antitumor effect of the sample against mouse mammary carcinoma MM46, the same test as described above was performed, and the results are shown in Table 7 below.

### TABLE 7

| Days of administration | Administration method | Tumor inhibitory ratio (%) | Average of survival days (days) | Increase of life span (%) |
|---|---|---|---|---|
| Control | — | — | 28.6±4.9 | 100 |
| Continuously for 20 days | Intraperi- toneously | 15.3 | 29.5±3.7 | 103.1 |
| At 10th and 14th days | Intratumorly | 52.0 | 29.9±7.4 | 104.5 |

Immunopotentiating activity

(a) Stimulatory activity on antibody formation

CDF$_1$ mice (8 weeks old, female) were immunized by intravenous injection of $1 \times 10^8$ of sheep red blood cells, and at the same time, Sample V was once administered intraperitoneally. At the 4th day from immunization, the number of antibody-forming cells (plaque-forming cells) in the spleen of mouse was measured by Jerne et al's method (Jerne, N. K. Nordin, A. A. & Henry, C.: *The Agar Plaque Technique for Recognizing Antibody-Producing Cells*, "Cell-Bound Antibodies", pp. 109 to 122 (1963)). The ratio (T/C) of the number of antibody-forming cells in the mouse to which the sample was administered against that in the mouse to which the sample was not administered is shown in Table 8 below.

### TABLE 8

| Administration amount | T/C (%) |
|---|---|
| 0 (No administration) | 100 |
| 0.1 microgram per mouse | 102 |
| 1 microgram per mouse | 118 |
| 10 micrograms per mouse | 181 |
| 100 micrograms per mouse | 245 |
| 1 milligram per mouse | 152 |

(b) Effect on delayed-type hypersensitivity reaction

Using CDF$_1$ mice (8 weeks old, female), the effect on the delayed-type hypersensitivity reaction to sheep red blood cells was examined according to Lagrange et al's method (Lagrange, P. H., Mackaness, G. B & Mieler, T.: *Potentiation of T-cell-mediated Immunity by Selective Suppression of Antibody Formation with Cyclophosphamide*, J. Exp. Med., Vol. 139, pp. 1529 to 1539 (1979)). The increased ratio of the delayed-type hypersensitivity reaction (ratio of the thickness of footpad of administered mouse to that of non-administered mouse), when Sample V was intraperitoneally administered is shown in Table 9.

### TABLE 9

| Administration amount | Increased ratio (%) |
|---|---|
| 0 (not administered) | 100 |
| 1.9 micrograms per mouse | 103 |
| 7.8 micrograms per mouse | 107 |
| 31.2 micrograms per mouse | 137 |
| 125 micrograms per mouse | 141 |
| 500 micrograms per mouse | 118 |

(c) Effect on incorporation of $^3$H-thymidine into spleen cells of mouse

The sample was added to spleen cells suspension obtained from $CDF_1$ mice (8 weeks old, female) and cultured at 37°C for 72 hours in the presence of 5% $CO_2$. The incorporation of $^3$H-thymidine into the cultured cell was examined.

The radioactivity when Sample V was added singly or in combination with concanavalin A (ConA) or endotoxin (LPS) was counted, and the results are shown in Table 10.

### TABLE 10

| Concentration of Sample V (micrograms per milliliter) | Count in the presence of Sample V alone (cpm) | Count in the presence of Sample V and ConA (0.5 micrograms per milliliter) (cpm) | Count in the presence of Sample V and LPS (2 micrograms per milliliter) (cpm) |
|---|---|---|---|
| 0 | 988 | 10318 | 5300 |
| 0.1 | 1232 | 12038 | 4683 |
| 1 | 2016 | 15680 | 6851 |
| 10 | 3758 | 22615 | 8654 |
| 100 | 16482 | 23743 | 13554 |

For Sample III, the same test as above was performed, and the results are shown in Table 11.

### TABLE 11

| Concentration of Sample III (micrograms per milliliter) | Count (cpm) | Increased ratio |
|---|---|---|
| 0 | 28 | — |
| 0.02 | 53 | 1.89 |
| 0.1 | 66 | 2.35 |
| 0.4 | 60 | 2.14 |
| 1.5 | 70 | 2.50 |
| 6.2 | 157 | 5.61 |
| 25 | 235 | 8.39 |
| 100 | 388 | 13.86 |

Effect on phagocytosis of macrophages

Sample V was administered intraperitoneally to $CDF_1$ mice (8 weeks old, female). In 18 hours after the

8

**0 053 800**

administration, macrophase in the peritoneal cavity was collected and incubated on a non-serum medium for 1 hour, and adherent cells were collected. The effect on phagocytosis of collected macrophages against yeast (*Saccharomyces cerevisiae*) was examined. The increased ratio of phagocytic ability of macrophages in administered mice to that of macrophages in non-administered mice is shown in Table 12.

TABLE 12

| Sample | Increased ratio (%) |
|---|---|
| 0 (not administered) | 100 |
| 0.6 microgram per mouse | 103 |
| 2.5 micrograms per mouse | 166 |
| 10 micrograms per mouse | 151 |
| 100 micrograms per mouse | 139 |

**Claims for the Contracting States: BE, CH, DE, FR, GB, IT, LI, LU, NL, SE**

1. A heteroglycan composed of (a) fucose, (b) mannose, and (c) glucose in the molar ratio of (a):(b):(c)=(0.15±0.05): (0.31±0.05):1, and having an infrared absorption spectrum as shown in Fig. 4.

2. A process for producing a heteroglycan as claimed in claim 1 which comprises cultivating Flavobacterium uliginosum MP-55 (FERM-P 5793) and recovering the heteroglycan produced and accumulated in nutrient media.

3. An immunopotentiator containing a heteroglycan as claimed in claim 1.

4. A heteroglycan as claimed in claim 1 for the use as a medicament.

5. A heteroglycan as claimed in claim 1 for the prevention and treatment of infectious diseases.

6. A heteroglycan as claimed in claim 1 for the use for treating tumors.

**Claims for the Contracting State: AT**

1. A process for producing a heteroglycan composed of (a) fucose, (b) mannose, and (c) glucose in the molar ratio of (a):(b):(c)=(0.15±0.05):(0.31±0.05):1 and having an infrared absorption spectrum as shown in Fig. 4 which comprises cultivating Flavobacterium uliginosum MP-55 (FERM-P 5793) and recovering the heteroglycan produced and accumulated in nutrient media.

2. A process for producing an immunopotentiator containing a heteroglycan as defined in claim 1 which process comprises bringing the same into a suitable form for the administration.

**Patentansprüche für die Vertragsstaaten: BE, CH, DE, FR, GB, IT, LI, LU, NL, SE**

1. Heteroglykan bestehend aus (a) Fucose, (b) Mannose und (c) Glucose im Molverhältnis (a):(b):(c)=(0,15±0,05):(0,31±0,05):1 mit dem in Fig. 4 abgebildeten Infrarotabsorptionsspektrum.

2. Verfahren zur Herstellung eines Heteroglykans nach Anspruch 1, dadurch gekennzeichnet, dass man Flavobacterium uliginosum MP-55 (FERM-P 5793) züchtet und das erzeugte, in den Nährmedien angereicherte Heteroglykan gewinnt.

3. Immunopotentiator, enthaltend ein Heteroglykan nach Anspruch 1.

4. Heteroglykan nach Anspruch 1 zur Verwendung als Arzneimittel.

5. Heteroglykan nach Anspruch 1 zur Verhütung und Behandlung von Infektionskrankheiten.

6. Heteroglykan nach Anspruch 1 zur Verwendung bei der Tumorbehandlung.

**Patentansprüche für den Vertragsstaat: AT**

1. Verfahren zur Herstellung eines Heteroglykans bestehend aus (a) Fucose, (b) Mannose und (c) Glucose im Molverhältnis (a):(b):(c)=(0,15±0,05):(0,31±0,05):1 und mit dem in Fig. 4 abgebildeten Infrarotabsorptionsspektrum, dadurch gekennzeichnet, dass man Flavobacterium uliginosum MP-55 (FERM-P 5793) züchtet und das erzeugte, in den Nährmedien angereicherte Heteroglykan gewinnt.

2. Verfahren zur Herstellung eines ein Heteroglykan nach Anspruch 1 enthaltenden Immunopotentiators, dadurch gekennzeichnet, dass man diesen in eine geeignete Verabreichungsform bringt.

**Revendications pour les Etats Contractants: BE, CH, DE, FR, GB, IT, LI, LU, NL, SE**

1. Hétéroglucane composé de (a) fucose, (b) mannose et (c) glucose, dans un rapport molaire (a):(b):(c) de (0,15±0,05):(0,31±0,05):(1) et ayant un spectre d'absorption infrarouge tel que représenté sur la Figure 4.

9

2. Procédé pour la production d'un hétéroglucane selon la revendication 1, qui consiste à cultiver Flavobacterium uliginosum MP-55 (FERM-P 5793) et à récupérer l'hétéroglucane produit et accumulé dans le milieu nutritif.

3. Immunopotentialisateur contenant un hétéroglucane selon la revendication 1.

4. Hétéroglucane selon la revendication 1, destiné à être utilisé comme médicament.

5. Hétéroglucane selon la revendication 1 pour la prévention et le traitement des maladies infectieuses.

6. Hétéroglucane selon la revendication 1, utilisable pour le traitement de tumeurs.

**Revendications pour l'Etat Contractant: AT**

1. Procédé pour la production d'un hétéroglucane composé de (a) fucose, (b) mannose et (c) glucose, dans un rapport molaire (a):(b):(c) de (0,15±0,05):(0,31±0,05):(1), et ayant un spectre d'absorption infrarouge tel que représenté sur la Figure 4, lequel procédé consiste à cultiver Flavobacterium uliginosum MP-55 (FERM-P 5793) et à récupérer l'hétéroglucane produit et accumulé dans le milieu nutritif.

2. Procédé pour la production d'un immunopotentialisateur contenant un hétéroglucane tel que défini dans la revendication 1, lequel procédé consiste à mettre l'hétéroglucane sous une forme d'administration appropriée.

FIG. 1

# FIG. 4

0 053 800